# EUROPEAN PATENT APPLICATION

(11) **EP 1 380 570 A1**
(43) Date of publication of application: **14.01.2004**
(21) Application number: 02717163.6
(22) Date of filing: 16.04.2002
(51) Int. Cl.: C07C 231/02, C07C 231/08, C07C 233/47, C07K 1/06, C07K 1/30, C07K 5/075, C12P 21/02

(54) **PROCESS FOR PRODUCING N-FORMYLAMINO ACID AND UTILIZATION THEREOF**

(30) Priority: 20.04.2001 JP 2001122345; 20.04.2001 JP 2001122346; 21.06.2001 JP 2001187540
(71) Applicant: Ajinomoto Co., Inc., Tokyo 104-8315 (JP)
(72) Inventor: KAWAHARA, Shigeru, Amino Science Lab., Kawasaki-shi, Kanagawa 210-0801 (JP); TAKEMOTO, Tadashi, Amino Science Lab., Kawasaki-shi,, Kanagawa 210-0801 (JP); FUJITA, Shinji, Amino Science Lab., Kawasaki-shi, Kanagawa 210-0801 (JP); OHNO, Ayako, Amino Science Lab., Kawasaki-shi, Kanagawa 210-0801 (JP)
(74) Representative: Nash, David Allan
(86) International application number: PCT/JP2002/003754
(87) International publication number: WO 2002/085840

(57) **Abstract**

Provided is a process for producing an N- formylamino acid important as various synthetic intermediates at good efficiency and industrially easily by reacting an amino acid such as aspartic acid or a neutral amino acid with formamide and/or methyl formate in the presence of a base, or by reacting a salt of the amino acid with formamide and/or methyl formate.

Further provided is a process for producing, at good efficiency and industrially easily, N-formyl-α-L-aspartyl-L-phenylalanine methyl ester as a precursor of a sweetener aspartame, and aspartame by further subjecting the ester to deformylation, in which in an enzyme condensation reaction of N-formyl-L-aspartic acid and/or its salt with L- and/or DL-phenylalanine methyl ester, an amount of water present in the reaction system is specified relative to the total weight amount of the reaction system.

It is also possible to provide a process for producing high-purity N-formyl-α-L-aspartyl-L-phenylalanine methyl ester by subjecting a phenylalanine methyl ester adduct of N-formyl-α-L-aspartyl-L-phenylalanine methyl ester formed to pH adjustment.

## Description

### Technical Field

The present invention relates to a novel process for producing an N-formyl-neutral-amino acid and N-formylaspartic acid. More specifically, it relates to a novel process for producing N-formyl-α-L-aspartyl-L-phenylalanine methyl ester as a precursor of aspartame and aspartame.

### Background Art

N-formylamino acids with an amino group protected with a formyl group, such as N-formylaspartic acid and N-formyl-neutral-amino acids are important compounds in the field of foods and pharmaceuticals as an intermediate for synthesis of various peptide compounds.

Especially, N-formylaspartic acid is an important compound as an intermediate of aspartame being a sweetener. A formyl group as a protecting group of an amino group of an amino acid can be introduced from a relatively inexpensive reagent, and it can be deprotected at low cost in comparison to, for example, a benzyloxycarbonyl group which is deprotected by reduction with palladium carbon. As a method for synthesizing N-formylaspartic acid, for example, a method is known in which, as shown in the following reaction scheme 1, aspartic acid is converted to N-formylaspartic anhydride using formic acid and acetic anhydride, followed by hydrolysis (refer to, for example, European Journal of Biochemistry, vol. 10, 318-323, 1969).

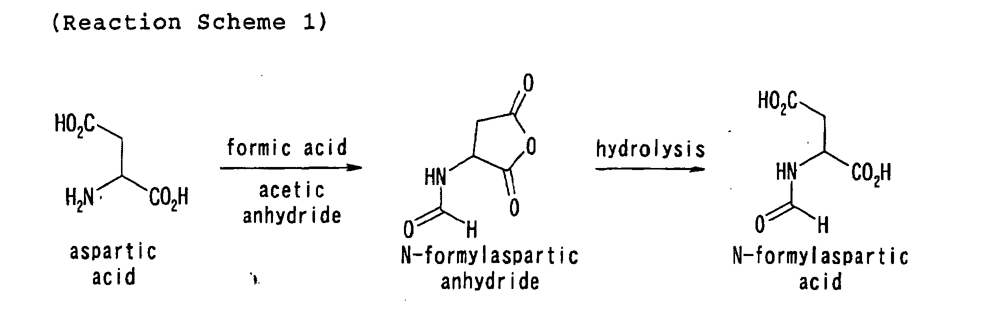

However, since this method uses large amounts of acetic anhydride and formic acid and requires the reaction with plural steps, it is not satisfactory as a method for producing N-formylaspartic acid.

Further, a method for synthesizing N-formyl-L-aspartic acid using formamide as shown in the following reaction scheme 2 is reported (refer to Example 1 of U. S. Patent No. 4,789,757).

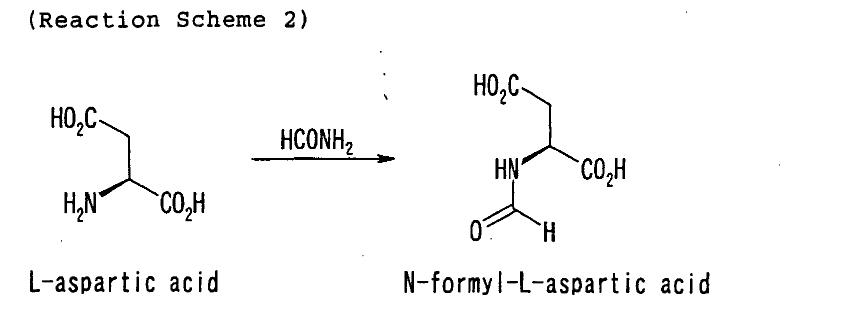

In Example 1 of U. S. Patent No. 4,789,757, it is described that N-formyl-L-aspartic acid is quantitatively formed by heat-stirring L-aspartic acid and formamide in an amount of 5 molar times that at from 95 to 100°C for 2 hours.

However, when the present inventors conducted a follow-up test of this Example 1, a yield of N-formyl-L-aspartic acid formed was approximately 40% relative to L-aspartic acid as a starting material. Thus, it can hardly be said that a method which is satisfactory enough as an industrial method for producing N-formylaspartic acid has been established.

Next, as a method for synthesizing N-formyl-α-L-aspartyl-L-phenylalanine methyl ester (hereinafter sometimes abbreviated as "F-APM") being a precursor of aspartame from N-formyl-L-aspartic acid (hereinafter sometimes abbreviated as "F-Asp"), a method in which L-phenylalanine methyl ester (hereinafter sometimes abbreviated as "L-PM") and F-Asp are reacted in an organic solvent has been proposed (refer to, for example, U. S. Patent No. 3,786,039).

In this method, however, an α-isomer and a β-isomer are both formed, and a separation-purification procedure for removing an unnecessary β-isomer is required. Thus, it is hardly said to be an industrially advantageous method.

As a method in which the foregoing isomer is not formed, for example, Japanese Patent Kokai Publication JP-A-60-164495 (EP 0149594) has reported a method in which F-APM is obtained by a condensation reaction of F-Asp and L-phenylalanine methyl ester in an aqueous solution in the presence of thermolysin as an enzyme. According to Example 1 thereof, F-Asp and L- PM are reacted at a molar ratio of 1:1, a slurry is separated, the resulting solid (which is identified to be a (1:1) adduct of N-formyl-α-L-aspartyl-L-phenylalanine methyl ester and L-phenylalanine methyl ester) is caused to float on water, a pH value is adjusted to 1.6, and F-APM is extracted with ethyl acetate. Nevertheless, the overall yield is approximately 12%. Further, in Example 3 thereof, F-Asp and L-PM are reacted at a molar ratio of 1:2, but a condensation yield is as low as 41% relative to F-Asp. In Example 2 thereof, F-Asp and L-PM are reacted at a molar ratio of 5:1, and a condensation yield is 90% relative to L-PM, but as low as 9% relative to F-Asp. Thus, the method is hardly said to be an industrially advantageous method.

Japanese Patent Kokai Publication JP-A-10-174597 discloses a method in which when synthesizing F-APM by a condensation reaction with an enzyme, a water-immiscible organic solvent having dissolved therein F-Asp and L-PM is continuously fed to an enzyme aqueous solution, and F-APM formed is extracted in an organic layer and continuously withdrawn. In this method, however, a method for isolating F-APM from the organic layer is not disclosed in the least, and it is thus unclear whether F-APM can finally be produced at good efficiency.

On the other hand, N-formyl-neutral-amino acids are, as stated above, important as an intermediate for synthesis of various peptide compounds. For example, N-formylleucine in which an amino group of leucine as a neutral amino acid is formylated has been used as a side chain of an appetite regulator, trade name "Orlistat", marketed by Roche. As a method for synthesizing an N-formylamino acid, a method in which an amino acid is formylated using formic acid and acetic anhydride (refer to Journal of American Chemical Society, 1958, vol. 80, p. 1154) has been known. In this method, however, large amounts of formic acid and acetic anhydride are used such that, for example, the amount of the former is 56 molar times and that of the latter 7 molar times the amount of the amino acid respectively. Accordingly, it is hardly said to be satisfactory as an industrial method for producing an N-formylamino acid.

A method for synthesizing an N-formylamino acid using formamide is proposed (refer to U. S. Patent No. 4,789,757). It is reported that an N-formylamino acid is quantitatively formed by heat- stirring an amino acid in formamide in an amount of 5 molar times that at from 95 to 100°C for 2 hours. However, the present inventors conducted a follow-up test of this method. Consequently, a yield of an N-formylamino acid formed was only approximately 40% or so. Accordingly, this method is also hardly said to be satisfactory as an industrial method for producing an N-formylamino acid.

Under these circumstances, a method for producing N-formylaspartic acid, N-formyl-α-L-aspartyl-L-phenylalanine methyl ester as a precursor of aspartame and an N-formyl-neutral-amino acid at good efficiency and industrially easily has been in demand.

### Problems that the Invention is to Solve

The problems that the invention is to solve reside in the developments of an efficient process for producing N-formylaspartic acid (or its salt) important as an intermediate for synthesis of a sweetener aspartame, an efficient process for producing N-formyl-α-L-aspartyl-L-phenylalanine methyl ester as a precursor of a sweetener aspartame and aspartame using the same as a starting material, and a process for producing an N-formyl-neutral-amino acid (or its salt) important as an intermediate for synthesis of a peptide compound efficiently and easily.

### Disclosure of the Invention

For solving the foregoing many problems, the present inventors have assiduously conducted investigations, and have consequently found a lot of the following new findings. On the basis of these findings, the present invention including the inventions of the following various embodiments has been completed.

### (New findings)

1. N-formylaspartic acid (or its salt) can be produced in a high yield by reacting aspartic acid with formamide and/or methyl formate in the presence of a base. N-formylaspartic acid (or its salt) can be produced in a high yield by reacting a salt of aspartic acid with formamide and/or methyl formate.
2. In a process for producing F-APM by an enzyme condensation reaction of F-Asp and/or its salt with L- and/or DL-phenylalanine methyl ester (hereinafter sometimes referred to as "L/DL-PM"), F-APM can be produced at good efficiency by specifying a concentration of F-Asp. Further, in the presence of a specific trialkyl phosphate, a desired compound F-APM is obtained in a slurry state and this compound is separated (by filtration or the like). In this manner, the desired compound can easily be obtained.
3. F-APM can be obtained by suspending an L- and/or D-phenylalanine methyl ester (hereinafter sometimes referred to as "L/D-PM") adduct of F-APM resulting from the foregoing enzyme condensation reaction in an aqueous solution having a pH value in a specific range and separating a solid.
4. An N-formyl-neutral-amino acid (or its salt) can be produced in a high yield by reacting a neutral amino acid with formamide and/or methyl formate in the presence of a base. An N-formyl-neutral-amino acid (or its salt) can be produced in a high yield by reacting a salt of a neutral amino acid with formamide and/or methyl formate.

### (Inventions of various embodiments included in the present invention)

The inventions of the following various embodiments included in the present invention are provided on the basis of the foregoing new findings.
1. A process for producing N-formylaspartic acid or its salt, characterized by reacting aspartic acid with formamide and/or methyl formate in the presence of a base; and
   a process for producing N-formylaspartic acid or its salt, characterized by reacting a salt of aspartic acid with formamide and/or methyl formate.
   The foregoing two are sometimes referred to as "formylation process 1 of the present invention".
2. A process for producing N-formyl-α-L-aspartyl-L-phenylalanine methyl ester (F-APM) (N-formyl-α-L-aspartyl-L-phenylalanine methyl ester may be in the form of an adduct with phenylalanine methyl ester), characterized in that in an enzyme condensation reaction of N-formyl-L-aspartic acid and/or its salt with L- and/or DL-phenylalanine methyl ester, a concentration of the N-formyl-L-aspartic acid in an aqueous solution is 1.2 mol/L or more; and
   a process for producing F-APM, wherein a trialkyl phosphate such as tri-n-butyl phosphate is caused to coexist in the enzyme condensation reaction, and the desired product (in the form of the adduct) obtained in a slurry state is separated.
   The foregoing two are sometimes referred to as "process for producing F-APM of the present invention; condensation reaction".
3. A prbcess for producing N-formyl-α-L-aspartyl-L-phenylalanine methyl ester, characterized by suspending a phenylalanine methyl ester adduct of N-formyl-α-L-aspartyl-L-phenylalanine methyl ester in an aqueous solution having a pH value of from 1.0 to 4.5, and separating a solid.
   The foregoing process is sometimes referred to as "process for producing F-APM of the present invention; separation method".
4. A process for producing an N-formyl-neutral-amino acid or its salt, characterized by reacting a neutral amino acid with formamide and/or methyl formate in the presence of a base; and
   a process for producing an N-formyl-neutral-amino acid or its salt, characterized by reacting a salt of a neutral amino acid with formamide and/or methyl formate.

The foregoing two are sometimes referred to as "formylation process 2 of the present invention".

### (For formylation process 1 of the present invention)

The invention lies in, as described above, a process for producing N-formylaspartic acid or its salt, which comprises
reacting aspartic acid with formamide and/or methyl formate in the presence of a base; or
reacting a salt of aspartic acid with formamide and/or methyl formate.

The type of the base used is not particularly limited. Examples of the base can include sodium hydroxide, potassium hydroxide, sodium carbonate, potassium carbonate and ammonia.

One or more of these can selectively be used.

The use amount of the base is not particularly limited. The base can be used in an amount of from 0.15 to 2.5 equivalents per equivalent of aspartic acid.

An optically active substance of aspartic acid is not particularly limited. Any of an L-form, a D-form and a DL-form can be used. L-aspartic acid can preferably be used as a precursor of aspartame.

It is advisable to use a reaction solvent. At this time, water, a polar solvent or a mixed solvent of water and a polar solvent can be used.

The use amount of formamide and/or methyl formate is not particularly limited. Formamide and/or methyl formate can be used in an amount of from 0.5 to 6 molar times that of aspartic acid.

In the invention, N-formylaspartic acid can be produced and obtained in the form of a free compound or a salt. For example, when it is required to obtain a free compound, the resulting salt of N-formylaspartic acid is subjected to a usual desalting step, whereby a free compound of it.can easily be produced and obtained. All these are included in the invention.

After N-formyl-L-aspartic acid (and/or its salt) is thus obtained, this N-formyl-L-aspartic acid (and/or its salt) and phenylalanine methyl ester (L-form, DL-form or the like) are condensed to form N-formyl-α-L-aspartyl-L-phenylalanine methyl ester which is then converted to aspartame. In this manner, aspartame can be produced. Such a process for producing N-formylaspartame (aspartame precursor) or aspartame is also included in the invention.

### (For process for producing F-APM of the present invention; condensation reaction)

The invention lies in a process for producing N-formyl-α-L-aspartyl-L-phenylalanine methyl ester, in which an enzyme condensation reaction of N-formyl-L-aspartic acid and/or its salt with L- and/or DL-phenylalanine methyl ester is conducted such that a concentration of the N-formyl-L-aspartic acid in an aqueous solution is 1.2 mol/L or more, preferably from 2.7 to 27 mol/L, more preferably from 3.5 to 17 mol/L. At this time, N-formyl-α-L-aspartyl-L-phenylalanine methyl ester may be in the form of an adduct with phenylalanine methyl ester.

In the enzyme reaction system, it is advisable to adjust a molar ratio of N-formyl-L-aspartic acid and/or its salt and L- and/or DL-phenylalanine methyl ester such that the latter is preferably from 0.5 to 3 or so, more preferably from 1.7 to 2.3 or so relative to 1 of N-formyl-L-aspartic acid.

The enzyme used is not particularly limited so long as it is an enzyme that catalyzes the enzyme condensation reaction of N-formyl-L-aspartic acid and L- and/or DL-phenylalanine methyl ester. A protease is preferably used.

With respect to N-formyl-L-aspartic acid and/or its salt used here, N-formyl-L-aspartic acid and/or its salt produced by the formylation process 1 of the present invention can preferably be used. Accordingly, it is preferable that the invention is performed subsequently to the formylation process 1 of the present invention.

As a trialkyl phosphate, a water-immiscible liquid trialkyl phosphate is used, and a trialkyl phosphate of which the three alkyl groups are, independently from each other, alkyl groups having from 4 to 6 carbon atoms is preferably used. Tri-n-butyl phosphate is especially preferable.

With respect to the use amount of the trialkyl phosphate, the trialkyl phosphate can be used at a weight ratio of, preferably from 2 to 30 or so, more preferably from 5 to 20 or so relative to 1 of a water solvent.

### (For process for producing F-APM of the present invention; separation method)

The invention lies in a process for producing N-formyl-α-L-aspartyl-L-phenylalanine methyl ester, which comprises suspending a phenylalanine methyl ester adduct of N-formyl-α-L-aspartyl-L-phenylalanine methyl ester in an aqueous solution having pH of from 1.0 to 4.5, preferably from 2 to 3 or so, and separating a solid.

As the adduct, the adduct obtained by the process for producing F-APM of the present invention; condensation reaction can be used. Accordingly, it is preferable that the process for producing F-APM of the present invention; separation method is performed subsequently to the process for producing F-APM of the present invention; condensation reaction.

As described above, after N-formyl-α-L-aspartyl-L-phenylalanine methyl ester is obtained, the formyl group is removed therefrom, whereby α-L-aspartyl-L-phenylalanine methyl ester (aspartame) can be produced. Such a process for producing aspartame is also included in the invention.

### (For formylation process 2 of the present invention)

The invention lies in a process for producing an N-formyl-neutral-amino acid or its salt, which comprises
reacting a neutral amino acid with formamide and/or methyl formate in the presence of a base; or
reacting a salt of a neutral amino acid with formamide and/or methyl formate.

The base used in the invention is not particularly limited. Examples thereof can include sodium hydroxide, potassium hydroxide, sodium carbonate, potassium carbonate and ammonia. One or more of these can selectively be used.

In the reaction, the base can be used in an amount of from 0.1 to 10 molar times or so that of the neutral amino acid.

The salt of the neutral amino acid used in the invention is not particularly limited. Examples thereof include a sodium salt, a potassium salt and an ammonium salt. One or more of these can selectively be used.

The type of the neutral amino acid used is not particularly limited. Examples of the neutral amino acid can include leucine, isoleucine and valine. One or more of these can be used as a starting material of the invention.

In the reaction, formamide and/or methyl formate can be used in an amount of from 0.5 to 6 molar times or so that of the neutral amino acid.

In the invention, the N-formyl-neutral-amino acid can be produced and obtained in the form of a free compound or a salt. For example, when it is required to obtain a free compound, the free compound can easily be produced and obtained by subjecting the resulting salt of N-formyl-neutral-amino acid to a usual desalting step. All these are included in the invention.

An optically active substance of the neutral amino acid used as the starting material is not particularly limited. Any of an L-form, a D-form and a DL-form can be used.

### Embodiments

The embodiments of the present invention are described in detail below. Since preferable typical examples are mainly described, the present invention is not limited thereto.

### (Formylation process 1 of the present invention)

The invention lies in
a process for producing N- formylaspartic acid or its salt, which comprises reacting aspartic acid with formamide and/or methyl formate in the presence of a base; and
a process for producing N-formylaspartic acid or its salt, which comprises reacting a salt of aspartic acid with formamide and/or methyl formate.

The optical isomerism of aspartic acid used in the invention is not particularly limited. Any of DL-aspartic acid, L-aspartic acid and D-aspartic acid can be used. In the production process of the present invention, racemization hardly occurs so long as the reaction is conducted under usual reaction conditions. By using optically active aspartic acid, N-formylaspartic acid or its salt can be obtained as an optically active substance.

It is advisable that the reaction in the invention is conducted in a solvent. However, the reaction can also be conducted in the absence of a solvent. The solvent is, when used, not particularly limited so long as it is inactive to the reaction. Water, a polar solvent or a mixed solvent of water and a polar solvent is preferably used.

The polar solvent is preferably a water-miscible polar solvent. Examples thereof include methanol, ethanol and dimethyl sulfoxide. These polar solvents may be used either singly or in combination.

The type of the base used is not particularly limited. Preferable examples thereof can include sodium hydroxide, potassium hydroxide, sodium carbonate, potassium carbonate and ammonia. Sodium hydroxide, potassium hydroxide and ammonia are especially preferable. These bases may be used either singly or in combination.

The salt of aspartic acid is not particularly limited. Examples thereof can include monosodium aspartate, disodium aspartate, monopotassium aspartate, dipotassium aspartate, monoammonium aspartate and diammonium aspartate. One or more of these can selectively be used.

When the solvent is used, the amount of the solvent used is not particularly limited so long as aspartic acid or its salt as a starting material and the base can be dissolved therein to such an extent that the effects of the invention are not impaired. Those skilled in the art can properly determine a preferable condition easily.

The use amount of formamide or methyl formate used is not particularly limited. Formamide or methyl formate can be used in an amount of, usually from 0.5 to 6 molar times or so, preferably from 1.5 to 3 molar times or so the amount of aspartic acid or its salt used. Incidentally, when the reaction is conducted in the absence of a solvent, it can be used in an amount of, usually from 2 to 10 molar times or so, preferably from 3 to 5 molar times or so.

Methyl formate and formamide can also be used in combination. In this case, the total use amount of formamide and methyl formate can be set in the foregoing range.

When the base is used, the amount of the base is usually from 0.2 to 10 equivalents or so, preferably from 0.3 to 5 equivalents or so per equivalent of aspartic acid used. By the way, "equivalent" here referred to is an acid-base equivalent. For example, in case of adding sodium hydroxide in an amount of 1 equivalent per equivalent of aspartic acid, 2 mol of sodium hydroxide is added when 1 mol of aspartic acid is present.

When the amount of the base is too small, a reaction rate tends to decrease, which is undesirable. Moreover, when the amount of the base is too large, a side reaction tends to proceed, which is undesirable.

The reaction temperature is not particularly limited, and it is usually from 30 to 130°C, preferably from 40 to 100°C. When the temperature is too high, a side reaction such as racemization tends to proceed. When the temperature is too low, a reaction rate tends to decrease. Thus, these are undesirable. It is advisable that the reaction is conducted with stirring.

In the invention, the base and aspartic acid cause a neutralization reaction to form a corresponding salt of aspartic acid. Accordingly, in the present invention, the salt of aspartic acid is used instead of aspartic acid, whereby the reaction can be conducted without adding the base. In this case, the same reaction conditions as described above may be used as the other reaction conditions. The optical isomerism of the salt of aspartic acid is not particularly limited either as described above. Any of a DL-aspartic acid salt, an L-aspartic acid salt and a D-aspartic acid salt can be used.

N-formylaspartic acid obtained by the invention is present in a solution in the form of the corresponding salt when completing the reaction. The reaction solution is optionally subjected to steps of neutralization, extraction, crystallization and the like in a usual manner, and a necessary desalting step or salt-forming step is utilized, whereby a desired product to be produced can be isolated and purified in the form of N-formylaspartic acid (free compound) or its salt.

N-formyl-α-L-aspartyl-L-phenylalanine methyl ester as an important intermediate (precursor) of aspartame can also be produced by enzymatically condensing the above-produced N-formyl-L-aspartic acid with L-phenylalanine methyl ester by a known method as described in, for example, Example 1 of Japanese Patent Kokoku Publication JP-B-60-164495. However, the above N-formyl-α-L-aspartyl-L-phenylalanine methyl ester can be obtained more efficiently by enzymatically condensing the same with L-phenylalanine methyl ester according to the process for producing F-APM of the present invention.

The thus-obtained N-formyl-α-L-aspartyl-L-phenylalanine methyl ester can be converted to aspartame by hydrolyzing the formyl group therein with a methanol-water mixed solvent in the presence of hydrochloric acid and then conducting neutralization thereto with sodium carbonate as described in the well-known method, for example, in Example 10 of Japanese Patent Kokai Publication JP-A-58-185545.

When the reaction is conducted especially at a low temperature in the process of the invention, crystals are sometimes precipitated during the reaction. In this case, it is advisable that the reaction is conducted while stirring the reaction solution relatively strongly. The stirring can be conducted with a stirrer such as a kneader or a homogenizer.

### (Process for producing F-APM of the present invention; condensation reaction)

The invention lies in a process for producing N-formyl-α-L-aspartyl-L-phenylalanine methyl ester (F-APM), characterized in that in an enzyme condensation reaction of N-formyl-L-aspartic acid and/or its salt (F-Asp) with L- and/or DL-phenylalanine methyl ester and/or their/its salt(s) (L/DL-PM) , a concentration of the F-Asp in an aqueous solution is 1.2 mol/L or more (The N-formyl-α-L-aspartyl-L-phenylalanine methyl ester may be in the form of an adduct with L- and/or D-phenylalanine methyl ester [hereinafter sometimes abbreviated as "F-APM^{·}L/D-PM"]).

By the way, N-formyl-L-aspartic acid in the enzyme condensation reaction of the present invention may be used in the form of a free compound or in the form of a salt such as a sodium salt. Likewise, L- and/or DL-phenylalanine methyl ester may be used in the form of a free compound or in the form of a salt such as a hydrochloride.

The concentration of F-Asp in the aqueous solution is preferably from 2.7 to 27 mol/L, more preferably from 3.5 to 17 mol/L. When the concentration of F-Asp in the aqueous solution is 1.2 mol/L or more, preferably from 2.7 to 27 mol/L, more preferably from 3.5 to 17 mol/L, the enzyme condensation reaction proceeds at a high conversion, whereby the yield of F-APM relative to F-Asp can be 50% or more, preferably 60% or more.

As the solvent of the enzyme condensation reaction, water alone is usually employed. It is also possible that water can be mixed with an organic solvent which is well miscible with water, such as methanol or ethanol unless it does not hinder the enzyme condensation reaction. However, it is usually unnecessary. Incidentally, the "aqueous solution" in the present invention includes not only a case in which the solvent in the enzyme condensation reaction is water alone but also a case in which the solvent is a mixed solvent of water and an organic solvent which is well miscible with water, such as methanol or ethanol.

A ratio of F-Asp to L/DL-PM in the aqueous solution in the enzyme condensation reaction is usually set in the range of from 1:0.5 to 1:3 in terms of a molar ratio. In the present invention, N-formyl-α-L-aspartyl-L-phenylalanine methyl ester as a reaction product is generally precipitated in the form of a (1:1) adduct with L- and/or D-phenylalanine methyl ester at the same time the enzyme condensation reaction proceeds (when the adduct is analyzed by HPLC, the respective peaks of F-Asp and L/D-PM are observed). Accordingly, for increasing the reaction yield, it is more preferable that the ratio is in the range of from 1:1.7 to 1:2.3 in terms of a molar ratio. Actually, the enzyme condensation reaction may be conducted on condition that the ratio is approximately 1:2 in terms of a molar ratio. When the enzyme condensation reaction is conducted at such a ratio to decrease an unreacted product, a complex purification step for removing the unreacted product can be simplified.

By the way, separately from water present at the outset of the reaction, water is generated as a byproduct when the condensation reaction proceeds. When the enzyme condensation reaction is conducted batchwise, the concentration of F-Asp and the ratio of F-Asp to L/DL-PM in the aqueous solution at the outset of the reaction can be set within the foregoing ranges. Further, when the enzyme condensation reaction is continuously conducted, the concentration of F-Asp and the ratio of F-Asp to L/DL-PM in the reaction vessel during the reaction can be set within the foregoing ranges.

As the enzyme, a neutral protease is preferably used. For example, a commercial thermolysin-like metalloprotease such as "Thermoase PS160" (made by Daiwa Kasei K.K.) or "Thermoase C160" (made by Daiwa Kasei K.K.) can be used. While the optimum pH value of the Thermoase is usually from 6 to 8 in the ordinary enzyme condensation reaction, the pH value in the enzyme condensation reaction of the present invention is set at, usually from 5 to 6, preferably from 5.2 to 5.8. When the enzyme condensation reaction is conducted at the pH value outside this range, a conversion is decreased. It is thus undesirable. Incidentally, the pH can be adjusted using a base such as sodium hydroxide, potassium hydroxide or sodium bicarbonate, or an acid such as hydrochloric acid or sulfuric acid.

The use amount of the enzyme can be usually from 0.025 to 4, preferably from 0.05 to 2 in terms of a weight ratio when the amount of F-Asp is defined as 1. In these enzymes, it is commonly known that the presence of a small amount of a calcium ion (II) is advantageously effective for stabilization and action of the enzyme. In the enzyme condensation reaction of the invention as well, it is preferable that the calcium ion (II) is present. As a reagent for the calcium ion (II) to be present in the reaction solution (in the aqueous solution), a calcium salt such as calcium carbonate, calcium chloride and calcium acetate can be used. The amount of the calcium ion (II) present in the aqueous solution is usually set in the range of from 0.15 to 0.5 based on an amount of enzyme used in terms of a weight ratio.

In the enzyme condensation reaction, the temperature can be set in the range of, usually from 10 to 60°C, preferably from 30 to 50°C. The reaction time is not particularly limited, but it can be usually set at from 30 minutes to 24 hours or so.

The reaction substrate is reacted at a high concentration in the enzyme condensation reaction of the present invention. Accordingly, as the reaction proceeds, the reaction solution usually loses a fluidity within the reaction vessel by the formation of the L- and/or D-phenylalanine methyl ester adduct of N-formyl-α-L-aspartyl-L-phenylalanine methyl ester, and it becomes solidified. However, the reaction solution can be rendered in a slurry state by conducting the enzyme condensation reaction in the presence of a water-immiscible specific trialkyl phosphate, and crystals of the adduct can be obtained efficiently by separating the solid (crystals of the adduct) from the slurry by a known separation method such as filtration or centrifugation. In this case, for rendering the solution in a slurry state, it is advisable to conduct the reaction with stirring. By the way, the enzyme condensation reaction proceeds well even in such a state that no dynamic action is exerted on the reaction system, for example, in a static state, so long as starting materials are mixed homogeneously enough at the outset of the reaction. Therefore, even though the fluidity is lost to make the stirring difficult, it does not pose a problem from the standpoint of a conversion of the enzyme reaction.

As the trialkyl phosphate used in the present invention, a water-immiscible liquid alkyl phosphate is used. A trialkyl phosphate of which the three alkyl groups are, independently from each other, alkyl groups having from 4 to 6 carbon atoms is preferably used. Especially, tri-n-butyl phosphate which can easily be procured is preferable. A water-miscible trialkyl phosphate such as trimethyl phosphate or triethyl phosphate is undesirable because it tends to deactivate the enzyme.

The use amount of the trialkyl phosphate is preferably from 2 to 30, more preferably from 5 to 20 in terms of a weight ratio relative to 1 of water used as a solvent of the enzyme condensation reaction. When a mixed solvent of water and an organic solvent well miscible with water is used as a solvent, the foregoing value can be set in terms of a weight ratio when the amount of water in the mixed solvent is defined as 1.

Separately from water present at the outset of the reaction, water is formed as a byproduct when the condensation reaction proceeds. In case the enzyme condensation reaction is conducted batchwise, the ratio of the trialkyl phosphate may be set on condition that the amount of water at the outset of the reaction is defined as 1. In case the enzyme condensation reaction is conducted continuously, it may be set on condition that the amount of water in the reaction vessel during the reaction is defined as 1.

Incidentally, since L- and/or DL-phenylalanine methyl ester tends to be transferred in a trialkyl phosphate phase during the enzyme condensation reaction, it is preferable that L- and/or DL-phenylalanine methyl ester (free compound) is previously dissolved in the trialkyl phosphate to be used from the standpoint of increasing the yield of the enzyme condensation reaction. The dissolution in a saturated state is more preferable. It is also possible that by anticipating an amount of L/DL-PM to be transferred in the trialkyl phosphate phase from the amount of the trialkyl phosphate present in the reaction system, the amount of L/DL-PM to be transferred therein can be previously dissolved additionally in the aqueous solution. However, L/DL-PM tends to be hydrolyzed in the aqueous solution. Further, in case of using a salt of L- and/or DL-phenylalanine methyl ester, an inorganic salt might be accumulated during the reaction and have an adverse effect on the enzyme reaction. Accordingly, from the standpoint of the reaction yield and the recovery and reuse of L/DL-PM, it is advisable that L/DL-PM is previously dissolved in the trialkyl phosphate. The trialkyl phosphate having L- and/or DL-phenylalanine methyl ester (free compound) dissolved therein or saturated therewith can be obtained by, for example, dissolving L/DL-PM in water and extracting it by addition of the trialkyl phosphate to separate an organic layer. At this time, it is advisable to conduct the extraction at a temperature of the enzyme condensation reaction which is actually performed. When the extraction is performed in this manner, the trialkyl phosphate might contain a certain amount of water. However, in the calculation of the concentration of F-Asp described above, the amount of water contained in this trialkyl phosphate is not included in the amount of water in the aqueous solution. By the way, the amount of water contained in the trialkyl phosphate can be measured by, for example, a Karl Fischer method, etc. When the trialkyl phosphate is saturated with L- and/or DL-phenylalanine methyl ester and water by the foregoing extraction operation at the actual reaction temperature, it is also possible that the amount of water (and L- and/or DL-phenylalanine methyl ester) contained in the saturated state is measured and water of amount which the trialkyl phosphate actually used is saturated with is additionally added to the aqueous solution in advance. At this time, the additional amount of water is not included in the amount of water in the aqueous solution in the foregoing calculation of the concentration' of F-Asp.

In the present invention, F-APM is usually obtained in the form of the 1:1 adduct with L-/D-PM. The free compound of F-APM can be obtained by the well-known separation method or the process for producing F-APM of the present invention; separation method to be described later. Further, the free compound can also be formed into an optional salt.

### (Process for producing F-APM of the present invention; separation method)

The invention lies in a process for producing N-formyl-α-L-aspartyl-L-phenylalanine methyl ester, characterized by suspending an L- and/or D-phenylalanine methyl ester adduct of N-formyl-α-L-aspartyl-L-phenylalanine methyl ester, for example, the 1:1 adduct thereof in an aqueous solution having a pH value of from 1.0 to 4.5, preferably from 2 to 3, and separating a solid. Incidentally, as the adduct (namely F-APM^{·}L/D-PM) used in the present invention, the adduct obtained by the foregoing condensation reaction in the present invention, preferably the adduct obtained as crystals by the foregoing condensation reaction carried out in the presence of the trialkyl phosphate can preferably be used.

The L- and/or D-phenylalanine methyl ester adduct of N-formyl-α-L-aspartyl-L-phenylalanine methyl ester (usually the 1:1 adduct thereof) as obtained by the enzyme condensation reaction of the present invention is suspended in the aqueous solution having the foregoing pH value to thereby dissolve L- and/or D-phenylalanine methyl ester in the aqueous solution. Accordingly, N-formyl-α-L-aspartyl-L-phenylalanine methyl ester can selectively be separated as a solid. The pH of the aqueous solution may previously be adjusted to the foregoing value either before suspending the adduct or after suspending the adduct. As the solvent of the aqueous solution, water alone is usually employed. An organic solvent well miscible with water, such as methanol or ethanol, can also be mixed with water. However, it is usually unnecessary. Incidentally, the "aqueous solution" here referred to includes not only a case in which the solvent is water alone, but also a case in which the solvent is a mixed solvent of water and an organic solvent well miscible with water, such as methanol or ethanol.

The amount of the aqueous solution is not particularly limited so long as the foregoing purpose is attained, and a skilled person in the art can determine a preferable condition as occasion demands. For example, it can be determined in the range of from 7 to 10 in terms of a weight ratio relative to 1 of the adduct. The temperature is not particularly limited either. The reaction can usually be conducted at room temperature. By the way, for further increasing the purity, this procedure may be repeated as required.

The thus-obtained N-formyl-α-L-aspartyl-L-phenylalanine methyl ester can be converted to α-L-aspartyl-L-phenylalanine methyl ester (aspartame) by a method known to those skilled in the art, for example, a method for removal of a formyl group (refer to, for example, Example 10 of Japanese Patent Kokai Publication JP-A-58-185545) in the presence of hydrochloric acid using a methanol-water mixed solvent.

Incidentally, in the production of aspartame, an arbitrary combination of any processes of "formylation process 1 of the present invention", "process for producing F-APM of the present invention; condensation reaction" (preferably the process performed in the presence of a trialkyl phosphate) and "process for producing F-APM of the present invention; separation method", preferably a combination of all the processes is used, whereby aspartame can be produced more efficiently and industrially easily.

### (Formylation process 2 of the present invention)

The invention lies in
a process for producing an N-formyl-neutral-amino acid or its salt, characterized by reacting a neutral amino acid with formamide and/or methyl formate in the presence of a base; and
a process for producing an N-formyl-neutral-amino acid or its salt, characterized by reacting a salt of a neutral amino acid with formamide and/or methyl formate.

A neutral amino acid or a salt of a neutral amino acid is used as a starting material of the invention. In a specific mode of the reaction, a neutral amino acid (free compound), a salt of a neutral amino acid or a mixture of these free compound and the salt thereof can be used as the starting material. Further, the neutral amino acid may be one type of an amino acid or a mixture of plural types of amino acids, and the salt may be one type of a salt or a mixture of plural types of salts. For industrially controlling the reaction, it is advisable to use one type of an amino acid or its salt (one type).

As stated earlier, the optical isomerism of the neutral amino acid used in the present invention is not particularly limited, and any of an L-form, a D-form and a DL-form may be used. In the present invention, racemization hardly occurs during the N-formylation reaction. An optically active neutral amino acid (or its salt) is used as the starting material, whereby the N-formyl-neutral-amino acid or its salt can be obtained as an optically active substance after the reaction.

With respect to the N-formylation reaction in the invention, an ordinary method known per se or a method to be developed in future as an amino group N-formylation method, other than the characterized part of the present invention, can be utilized.

The formylation reaction in the invention may be conducted in the absence of a solvent. A solvent, when used, is not particularly limited so long as it is inactive to the reaction. Water, a polar solvent or a mixed solvent of water and a polar solvent is preferably used.

The polar solvent is preferably a water-miscible solvent. Examples thereof include alcohols such as methanol and ethanol, and dimethyl sulfoxide. Of course, these polar solvents may be used in combination.

The type of the base used is not particularly limited. Hydroxides such as sodium hydroxide and potassium hydroxide, carbonates such as sodium carbonate and potassium carbonate, ammonia and amines such as triethylamine are preferably used. Especially, sodium hydroxide, potassium hydroxide, ammonia and the like are preferable. of course, these bases may be used either singly or in combination.

When the solvent is used, the amount of the solvent used is not particularly limited so long as it can dissolve the neutral amino acid (or its salt) as the starting material and the base to such an extent that the effects of the invention are not impaired. Any skilled person in the art can selectively determine a preferable condition easily as occasion demands.

The use amount of formamide or methyl formate to be used is not particularly limited. It is preferably from 0.5 to 6 molar times or so, more preferably from 1.5 to 3 molar times or so the amount of the neutral amino acid or its salt used as a starting material, or the mixture when the free compound and the salt are used in the form of the mixture as a starting material. Incidentally, when the reaction is conducted in the absence of the solvent, it can be used in an amount of, preferably from 2 to 10 molar times or so, more preferably from 3 to 5 molar times or so. As the foregoing formylating agent, the mixture of formamide and methyl formate can also be used. In this case, this mixture can be used in an amount of, preferably from 0.5 to 6 molar times or so, more preferably from 1.5 to 3 molar times or so the amount of the starting material. Further, when the reaction is conducted in the absence of the solvent, it can be used in an amount of, preferably from 2 to 10 molar times or so, more preferably from 3 to 5 molar times or so.

With respect to the use amount of the base in the invention, the base can be used in an amount of, preferably from 0.1 to 10 molar times or so, more preferably from 0.2 to 5 molar times or so, further preferably from 0.2 to 2.5 molar times or so the amount of the neutral amino acid as a starting material.

When the amount of the base is too small, the reaction rate tends to decrease, which is undesirable. When the amount of the base is too large, a side reaction tends to proceed, which is undesirable.

The reaction temperature used in the invention is not particularly limited. When it is too high, a problem of a side reaction such as racemization occurs. When it is too low, the reaction tends to be retarded. Accordingly, it can be selected from the range of, preferably from 30 to 130°C or so, more preferably from 40 to 100°C or so.

In the invention, the neutral amino acid (free compound) can be used as the starting material, and the salt of the neutral amino acid can also be used. In the specific mode of the reaction, the mixture thereof can also be used. Even when the salt of the neutral amino acid is used as the starting material, the reaction conditions and the like are the same as mentioned above, and the reaction can easily be carried out from the foregoing description. Incidentally, when the neutral amino acid in the form of the free compound is used as the starting material in the present invention, the base and the neutral amino acid cause neutralization in the reaction to form the corresponding salt. Accordingly, the thus-formed salt can be designated as the salt of a neutral amino acid as the starting material, or the salt of the neutral amino acid separately produced or prepared as the starting material can be designated as the salt of a neutral amino acid.

Examples of the salt of the neutral amino acid can include a sodium salt, a potassium salt and an ammonium salt.

The N-formyl-neutral-amino acid obtained by the present invention is usually present in the form of the corresponding salt when the reaction is completed. The desired product to be produced can be obtained in the form of the N-formyl-neutral-amino acid (free compound) or its salt by optionally subjecting the reaction solution to steps of neutralization, extraction, crystallization and the like in a usual manner and by utilizing a necessary desalting step or salt-forming step.

### Preferred Embodiments

The present invention is illustrated specifically below by referring to Examples, Comparative Examples and Reference Examples. However, the present invention is not limited to these Examples.

### (Formylation process 1 of the present invention)

### <Example 1>

1.0 g (7.5 mmol) of L-aspartic acid, 0.30 ml (7.5 mmol) of formamide and 0.21 ml of water were added to a reaction vessel sequentially, and 0.78 ml (9.6 M, 7.5 mmol) of a sodium hydroxide aqueous solution was then added thereto to adjust pH to 8.7. After the reaction was conducted at 50°C for 22 hours, the product was analyzed by high-performance liquid chromatography (HPLC). As a result, N-formyl-L-aspartic acid was formed in an amount of 0.45 g (36.8%; based on L-aspartic acid).

### <Example 2>

1.02 g (7.7 mmol) of L-aspartic acid, 0.61 ml (15.0 mmol) of formamide and 0.19 ml of water were added to a reaction vessel sequentially, and 0.80 ml (9.6 M, 7.7 mmol) of a sodium hydroxide aqueous solution was then added thereto to adjust pH to 8.4. After the reaction was conducted at 50°C for 22 hours, the product was analyzed by high-performance liquid chromatography. As a result, N-formyl-L-aspartic acid was formed in an amount of 0.42 g (33.9%; based on L-aspartic acid).

### <Example 3>

1.00 g (7.5 mmol) of L-aspartic acid, 0.91 ml (22.5 mmol) of formamide and 0.21 ml of water were added to a reaction vessel sequentially, and 0.78 ml (9.6 M, 7.5 mmol) of a sodium hydroxide aqueous solution was then added thereto to adjust pH to 8.7. After the reaction was conducted at 50°C for 22 hours, the product was analyzed by high-performance liquid chromatography. As a result, N-formyl-L-aspartic acid was formed in an amount of 0.59g (48.7%; based on L-aspartic acid).

### <Example 4>

1.03 g (7.7 mmol) of L-aspartic acid, 0.30 ml (7.5 mmol) of formamide and 0.77 ml of water were added to a reaction vessel sequentially, and 0.61 g (15.4 mmol) of pellet type sodium hydroxide was then added thereto to adjust pH to 11.1. After the reaction was conducted at 50°C for 22 hours, the product was analyzed by high-performance liquid chromatography. As a result, N-formyl-L-aspartic acid was formed in an amount of 0.76 g (61.6%; based on L-aspartic acid).

### <Example 5>

1.01 g (7.6 mmol) of L-aspartic acid, 0.61 ml (15.0 mmol) of formamide and 0. 77 ml of water were added to a reaction vessel sequentially, and 0.57 g (15.1 mmol) of pellet type sodium hydroxide was then added thereto to adjust pH to 11.2. After the reaction was conducted at 50°C for 22 hours, the product was analyzed by high-performance liquid chromatography. As a result, N-formyl-L-aspartic acid was formed in an amount of 1.1 g (90.6%; based on L-aspartic acid).

### <Example 6>

1.02 g (7.7 mmol) of L-aspartic acid, 0.91 ml (22.5 mmol) of formamide and 0.77 ml of water were added to a reaction vessel sequentially, and 0.61 g (15.4 mmol) of pellet type sodium hydroxide was then added thereto to adjust pH to 11.2. After the reaction was conducted at 50°C for 22 hours, the product was analyzed by high-performance liquid chromatography. As a result, N-formyl-L-aspartic acid was formed in an amount of 1.2 g (94.4%; based on L-aspartic acid).

### <Example 7>

1.50 g (8.8 mmol) of L-aspartic acid monopotassium salt and 1.20 g (26.3 mmol) of formamide were added to a reaction vessel, and then reacted at 100°C for 4.5 hours. When the product was analyzed by high-performance liquid chromatography, N-formyl-L-aspartic acid was formed in an amount of 1.20 g (85.0%; based on L-aspartic acid).

### <Example 8>

1.0 g (7.6 mmol) of L-aspartic acid, 0.71 g (15.2 mmol) of formamide and 1.11 g (19.0 mmol) of 29% aqueous ammonia were added to a reaction vessel in this order, and then reacted at 50°C for 27 hours. When the product was analyzed by high-performance liquid chromatography, N-formyl-L-aspartic acid was formed in an amount of 1.1 g (88.8%; based on L-aspartic acid).

### <Example 9>

10.0 g (75.1 mmol) of L-aspartic acid, 7.0 g (150.3 mmol) of formamide and 21.4 ml of 30% methanol-water were added to a reaction vessel in this order, and 6.26 g (150.3 mmol) of pellet type sodium hydroxide was then added thereto to adjust pH to 10.4. After the reaction was conducted at 40°C for 27 hours, the product was analyzed by high-performance liquid chromatography. As a result, N-formyl-L-aspartic acid was formed in an amount of 9 .4 g (77.7%; based on L-aspartic acid).

### <Comparative Example 1>

According to Example 1 of US 4789757, 1.0 g (7.7 mmol) of L-aspartic acid and 1.8 g (38.4 mmol) of formamide were added to a reaction vessel, and reacted at 95°C for 2 hours. When the product was analyzed by high-performance liquid chromatography, N-formyl-L-aspartic acid was formed in an amount of 0.49 g (39.8%; based on L-aspartic acid).

### <Comparative Example 2>

1.0 g (7.5 mmol) of L-aspartic acid, 0.91 ml (22.5 mmol) of formamide and 1. 0 ml of water were added to a reaction vessel sequentially, and then reacted at 50°C for 22 hours. When the product was analyzed by high-performance liquid chromatography, N-formyl-L-aspartic acid was formed in an amount of 0.015 g (1.2%; based on L-aspartic acid).

### <Example 10>

19.9 g (0.15 mol) of L-aspartic acid, 12.1 g (0.30 mol) of pellet type sodium hydroxide and 30 ml of water were added to a reaction vessel, and cooled to 0°C. 27.3 g (0.45 mol) of methyl formate was added dropwise thereto over a period of 9 hours, and the mixture was then vigorously stirred at 0°C for 11 hours. When the product was analyzed by high-performance liquid chromatography, N-formyl-L-aspartic acid was formed in an amount of 16.6 g (68.9%; based on L-aspartic acid).

### <Example 11>

19.9 g (0.15 mol) of L-aspartic acid, 12.1 g (0.30 mol) of pellet type sodium hydroxide and 60 ml of water were added to a reaction vessel, and cooled to 0°C. 27.3 g (0.45 mol) of methyl formate was added dropwise thereto over a period of 9 hours, and the mixture was then vigorously stirred at 0°C for 12 hours. When the product was analyzed by high-performance liquid chromatography, N-formyl-L-aspartic acid was formed in an amount of 12.6 g (52.1%; based on L-aspartic acid).

### <Example 12>

19.9 g (0.15 mol) of L-aspartic acid, 12.1 g (0.30 mol) of pellet type sodium hydroxide and 15 ml of water were added to a reaction vessel, and cooled to 0°C. 27.8 g (0.45 mol) of methyl formate was added dropwise thereto over a period of 9 hours, and the mixture was then vigorously stirred at 0°C for 12 hours. When the resulting slurry was analyzed by high-performance liquid chromatography, N-formyl-L-aspartic acid was formed in an amount of 17.7 g (73.2%; based on L-aspartic acid).

### <Example 13>

19.9 g (0.15 mol) of L-aspartic acid, 12.1 g (0.30 mol) of pellet type sodium hydroxide and 30 ml of water were added to a reaction vessel, and the temperature was adjusted to 25°C. 27.3 g (0.45 mol) of methyl formate was added dropwise thereto over a period of 1 hour, and the mixture was then vigorously stirred at 25°C for 13 hours. When the resulting reaction solution was analyzed by high-performance liquid chromatography, N-formyl-L-aspartic acid was formed in an amount of 13.5 g (56.0%; based on L-aspartic acid).

### <Example 14>

A bench kneader manufactured by Irie Shokai was used as a stirrer.

99.7 g (0.75 mol) of L-aspartic acid, 60.4 g (1.45 mol) of pellet type sodium hydroxide and 151 ml of water were added to the kneader, and the temperature was adjusted to 0°C. 136.4 g (2.20 mol) of methyl formate was added dropwise thereto over a period of 9 hours, and the mixture was then vigorously stirred at 0°C for 2 hours. When the resulting slurry was analyzed by high-performance liquid chromatography, N-formyl-L-aspartic acid was formed in an amount of 84.2 g (69.8%; based on L-aspartic acid).

### <Example 15>

19.9 g (0.15 mol) of L-aspartic acid, 19.8 g (content 85%, 0.30 mol) of pellet type potassium hydroxide and 30 ml of water were added to a reaction vessel, and the temperature was adjusted to 25°C. 27.3 g (0.45 mol) of methyl formate was added dropwise thereto over a period of 1 hour, and the mixture was then vigorously stirred at 25°C for 13 hours. When the resulting reaction solution was analyzed by high-performance liquid chromatography, N-formyl-L-aspartic acid was formed in an amount of 9.91 g (41%; based on L-aspartic acid).

### <Example 16>

10.0 g (75.1 mmol) of L-aspartic acid and 28.4 g (480.8 mmol) of 29% aqueous ammonia were added to a reaction vessel, and the temperature was adjusted to 25°C. 9.2 g (150.3 mmol) of methyl formate was added thereto over a period of 5 minutes, and the mixture was then stirred at 50°C for 24 hours. When the resulting reaction solution was analyzed by high-performance liquid chromatography, N-formyl-L-aspartic acid was formed in an amount of 9.4 g (77.9%; based on L-aspartic acid).

### <Example 17>

19.9 g (0.15 mol) of L-aspartic acid, 12.1 g (0.30 mol) of pellet type sodium hydroxide and 40 ml of 20% methanol-water were added to a reaction vessel, and the temperature was adjusted to 25°C. 27.3 g (0.45 mol) of methyl formate was added dropwise thereto over a period of 1 hour, and the mixture was then vigorously stirred at 25°C for 13 hours. When the resulting reaction solution was analyzed by high-performance liquid chromatography, N-formyl-L-aspartic acid was formed in an amount of 10.2 g (42.2%; based on L-aspartic acid).

### <Comparative Example 3>

19.9 g (0.15 mol) of L-aspartic acid and 30 ml of water were added to a reaction vessel, and the temperature was adjusted to 25°C. 27.3 g (0.45 mol) of methyl formate was added thereto, and the mixture was then stirred as such for 48 hours. When the resulting reaction solution was analyzed by high-performance liquid chromatography, N-formyl-L-aspartic acid was not formed at all.

### <Comparative Example 4>

According to Example 1 described in U. S. Patent No. 4,789,757, 1.01 g (7.7 mmol) of L-aspartic acid and 1.55 ml (38.4 mmol) of formamide were added to a reaction vessel, and then reacted at 95°C for 2 hours. When the product was analyzed by high-performance liquid chromatography, N-formyl-L-aspartic acid was formed in an amount of 0.49 g (39.8%; based on L-aspartic acid).

### (Process for producing F-APM of the present invention; condensation reaction)

A conversion ratio of an enzyme condensation reaction was measured by so-called high-performance liquid chromatography (HPLC) with an ultraviolet spectroscopic detector of 210 nm on a column filled with an octadecyl group-bound silica gel using a 0.1 M potassium dihydrogenphosphate aqueous solution (pH=2.8)/acetonitrile solvent.

### <Example 18>

L-phenylalanine methyl ester hydrochloride (2.16 g: 10.0 mmol), 2.11 g of sodium N-formyl-L-aspartate (containing 0.81 g (5.0 mmol) of N-formyl-L-aspartic acid) , 65 mg of calcium chloride dihydrate and 1.6 g of water were mixed and dissolved in a 20-ml egg plant-type flask at 40°C. Subsequently, the mixed aqueous solution was adjusted to a pH value of 5.45 by addition of a 25 wt% sodium hydroxide aqueous solution. Water was distilled off under reduced pressure to adjust the water content in the mixed aqueous solution to 17.5 wt% (concentration of N-formyl-L-aspartic acid 7.2 mol/L). Then, the remaining clear solution was mixed with 0.30 g of crude thermolysin (powder made by Daiwa Kasei K.K.: trade name "Thermoase PS160", containing approximately 40% of a protease protein and approximately 60% of sodium sulfate). After the reaction at 40°C for 20 hours, the reaction solution became solid. The solid matter was dissolved in water, and analysed by HPLC. Consequently, it was identified that a final conversion ratio to F-APM reached 85% based on N-formyl-L-aspartic acid.

### <Example 19>

The same operation as in Example 18 was conducted except that the water content in the mixed aqueous solution af ter water was distilled off was changed to 30 wt% (concentration of N-formyl-L-aspartic acid 3.6 mol/L) in Example 18. Consequently, it was identified that a final conversion ratio to F-APM reached 62.9% based on F-Asp.

### <Example 20>

0. 66 g of a 10 wt% calcium chloride aqueous solution and 1.0 g of water were added to L-phenylalanine methyl ester hydrochloride (1.08 g: 5.0 mmol) and N-formyl-L-aspartic acid (0.81 g: 5.0 mmol), and these were dissolved at 40°C. The solution was adjusted to a pH value of 5.8 with a 15 wt% sodium hydroxide aqueous solution. Water was distilled off under reduced pressure to adjust the water content of the mixed aqueous solution to 17.5 wt% (concentration of N-formyl-L-aspartic acid 10.8 mol/L). Subsequently, 0.30 g of crude thermolysin (the same "Thermoase PS160" as used in Example 18) was added thereto, and the reaction solution was stirred such that it became homogeneous. Af ter the reaction at 40°C for 20 hours, the reaction solution became solid (in a solid state). The solid matter was dissolved in water, and analyzed by HPLC. Consequently, it was identified that the yield of the L-PM adduct reached 41.7% based on N-formyl-L-aspartic acid. This shows that a conversion ratio to F-APM is 83.4%.

### <Example 21>

The same condensation reaction was conducted as in Example 20 except that the amount of L-phenylalanine methyl ester hydrochloride was changed to 0.54 g (2.5 mmol) in Example 20. When analysis was performed by HPLC, it was identified that the yield of the L-PM adduct reached 34.8% based on N-formyl-L-aspartic acid. This shows that a conversion ratio to F-APM is 69.6%.

### <Comparative Example 5>

0.71 g of a 10 wt% calcium chloride aqueous solution and 1.0 g of water were added to 2.16 g (10.0 mmol) of L-phenylalanine methyl ester hydrochloride and 2.11 g of sodium N-formyl-L-aspartate (containing 0.81 g (5.0 mmol) of N-formyl-L-aspartic acid), and the solution was adjusted to a pH value of 5.5 with a 25 wt% sodium hydroxide aqueous solution. Further, the total amount was adjusted to 8.62 g with water (water content in the mixed aqueous solution 59.0% by weight) (concentration of N-formyl-L-aspartic acid 1.0 mol/L). This solution was mixed with 0.30 g of crude thermolysin (the same "Thermoase PS160" as used in Example 18). After the reaction at 40°C for 20 hours, a solid precipitated in the reaction was dissolved in water, and analyzed by HPLC. Consequently, it was identified that a final conversion ratio to F-APM was 45.7% based on F-Asp.

### <Reference Example 1>

0.7 g of a 10% by weight calcium chloride aqueous solution and 1.0 g of water were added to 2.15 g (10.0 mmol) of L-phenylalanine methyl ester hydrochloride and 2.11 g of sodium F-N-formyl-L-aspartate (containing 0.81 g (5.0 mmol) of F-Asp), and the solution was adjusted to a pH value of 6.5 with a 25 wt% sodium hydroxide aqueous solution. Water was distilled off under reduced pressure to adjust the water content in the mixed aqueous solution to 18.7 wt% (concentration of N-formyl-L-aspartic acid 6.6 mol/L). Subsequently, this was mixed with 0.30 g of crude thermolysin (the same "Thermoase PS160" as used in Example 18). After the reaction at 40°C for 18 hours, the reaction solution became solid (in a solid state). The solid matter was dissolved in water, and analyzed by HPLC. Consequently, a final conversion ratio to F-APM is 43.0% based on F-Asp.

### <Reference Example 2>

The same condensation reaction was conducted as in Reference Example 1 except that the pH value was adjusted to 7.5 in Reference Example 1. A final conversion ratio to F-APM was 5.8% based on F-Asp.

### (Process for producing F-APM of the present invention; condensation reaction: presence of trialkyl phosphate)

### <Example 22>

L-phenylalanine methyl ester hydrochloride (8.61 g: 40 mmol) was dissolved in 20.0 g of water, and 20.0 g of tri-n-butyl phosphate (hereinafter abbreviated as "TBP") was added thereto. The mixture was vigorously stirred, and adjusted to a pH value of 7.0 with a 15 wt% sodium hydroxide aqueous solution. The reaction solution was further vigorously stirred at 40°C for 30 minutes, extracted, and then separated into an aqueous layer and an organic layer. This organic layer was used in the following reaction as an L-PM/TBP solution (PM: 1.05 mmol/g; 7% by weight).

0.66 g of a 10 wt% calcium chloride aqueous solution and 1.0 g of water were added to L-phenylalanine methyl ester hydrochloride (2.15 g: 10 mmol) and N-formyl-L-aspartic acid (0.81 g: 5.0 mmol), and these were dissolved at 40°C. The solution was adjusted to a pH value of 5.8 with a 15 wt% sodium hydroxide aqueous solution. Water was distilled off under reduced pressure to adjust the water content in the mixed aqueous solution to 23.3% (concentration of N-formyl-L-aspartic acid 5.05 mol/L). Subsequently, 12 g of a PM/TBP solution (TBP 8.9 g) and 0.3 g of crude thermolysin (powder made by Daiwa Kasei K.K.; trade name "Thermoase PS160", containing approximately 40% of a protease protein and approximately 60% of sodium sulfate) were added thereto, and the reaction solution was stirred such that it became homogeneous. After stirring was conducted at 40°C for 20 hours, the resulting slurry was separated by filtration to obtain crystals of an L-PM adduct of F-APM. The crystals and the mother liquor were analyzed by HPLC respectively. Consequently, it was identified that a final conversion ratio to F-APM reached 77.3% (73.3% in the crystals and 4.0% in the mother liquor) based on F-Asp.

### <Example 23>

The enzyme condensation reaction was conducted in the same manner as in Example 22 except that 6.0 g of the PM/TBP solution (TBP 4.45 g)was added. The resulting slurry was separated by filtration, and the crystals and the mother liquor were analyzed by HPLC. Consequently, a final conversion ratio to F-APM was 72.0% (68.4% in the crystals and 3.6% in the mother liquor).

### <Example 24>

The enzyme condensation reaction was conducted in the same manner as in Example 22 except that 16.6 g of the PM/TBP solution (TBP 12.32 g) was added. The resulting slurry was separated by filtration, and the crystals and the mother liquor were analyzed by HPLC. Consequently, a final conversion ratio to F-APM was 54.1% (47.3% in the crystals and 6.8% in the mother liquor).

### <Example 25>

The enzyme condensation reaction was conducted in the same manner as in Example 22 except that the concentration of N-formyl-L-aspartic acid was changed to 6.25 mol/L. The resulting slurry was separated by filtration, and the crystals and the mother liquor were analyzed by HPLC. Consequently, a final conversion ratio to F-APM was 65.1% (62.8% in the crystals and 2.3% in the mother liquor).

### <Example 26>

The enzyme condensation reaction was conducted in the same manner as in Example 22 except that the concentration of N-formyl-L-aspartic acid was changed to 3.58 mol/L. The resulting slurry was separated by filtration, and the crystals and the mother liquor were analyzed by HPLC. Consequently, it was identified that a final conversion ratio to F-APM was 58.2% (54.9% in the crystals and 3.3% in the mother liquor).

### <Example 27>

The enzyme condensation reaction was conducted in the same manner as in Example 22 except that the concentration of N-formyl-L-aspartic acid was changed to 1.53 mol/L. The resulting slurry was separated by filtration, and the crystals and the mother liquor were analyzed by HPLC. Consequently, a final conversion ratio to F-APM was 54.6% (49.0% in the crystals and 5.6% in the mother liquor).

### (Process for producing F-APM of the present invention; separation method)

### <Example 28>

The same reaction as in Example 18 was conducted. To the resulting reaction solution was added 10 ml of water to disperse the precipitated solid. The dispersion was stirred for 1 hour with ice cooling, and separated into a solid and a liquid. Further, wet crystals were washed with 2 ml of water. When 2.73 g of the resulting wet crystals was analyzed by HPLC, 42.4% by weight (3.59 mmol) of F-APM and 25.1% by weight (3.82 mmol) of L-PM were contained therein. Further, 1.0% by weight (0.42 mmol) of F-APM was contained in 13.4 g of the mother liquor containing the crystal wash liquid. That is, the total reaction yield is 80%, and the yield after the separation is 72%. The wet crystals obtained herein were suspended in 10 ml of water, and a pH value was adjusted to 1.7 with 1 N hydrochloric acid. 1.86 g of wet crystals was obtained by separation of the suspension after stirring for 1 hour with ice cooling. The wet crystals contained 54.0% by weight (3.11 mmol) of F-APM and 2.9% by weight (0.3 mmol) of L-PM. That is, it was identified that the yield of F-APM was 86.8% and 91.4% of L-PM was selected in the mother liquor.

### <Example 29>

The same operation as in Example 22 was conducted to obtain 4.98 g of wet crystals of an L-PM adduct of F-APM (F-APM: 3.64 mmol, L-PM: 4.62 mmol, reaction yield: 77.4%, yield of F-APM in crystals 72.8%). The resulting wet crystals were suspended in 10 ml of water, and then cooled with ice. The suspension was adjusted to a pH value of 1.8 with 1 N hydrochloric acid. The resulting suspension was stirred for 1 hour with ice cooling, and filtered to obtain 1.92 g of wet crystals. The wet crystals contained 3.17 mmol of F-APM and 0.43 mmol of L-PM. That is, it was identified that a yield of F-APM was 87.1% and 93% of L-PM was selected in the mother liquor.

### (Formylation process 2 of the present invention)

### <Example 30>

1.31 g (0.01 mol) of L-leucine, 0.40 g (0.01 mol) of sodium hydroxide, 1.35 g (0.03 mol) of formamide and 1.2 ml of water were added to a reaction vessel, and reacted at 50°C for 48 hours. With respect to the resulting reaction solution, the product was analyzed by high-performance liquid chromatography. Consequently, N-formyl-L-leucine was formed in an amount of 1.18 g (74.1% based on L-leucine).

### <Example 31>

1.31 g (0.01 mol) of L-leucine, 0.64 g (0.011 mol) of 29% aqueous ammonia, 1.35 g (0.03 mol) of formamide and 1.2 ml of water were added to a reaction vessel, and reacted at 50°C for 48 hours. With respect to the resulting reaction solution, the product was analyzed by high-performance liquid chromatography. Consequently, N-formyl-L-leucine was formed in an amount of 1.14 g (71.6% based on L-leucine).

### <Example 32>

1.31 g (0.01 mol) of L-leucine, 0.40 g (0.01 mol) of sodium hydroxide, 0.90 g (0.02 mol) of formamide and 1.2 ml of water were added to a reaction vessel, and reacted at 80°C for 5 hours. With respect to the resulting reaction solution, the product was analyzed by high-performance liquid chromatography. Consequently, N-formyl-L-leucine was formed in an amount of 1.11 g (69.7% based on L-leucine).

### <Example 33>

1.31 g (0.01 mol) of L-leucine, 0.40 g (0.01 mol) of sodium hydroxide, 1.80 g (0.03 mol) of methyl formate and 1.2 ml of water were added to a reaction vessel, and reacted under ice cooling for 2 hours and then at room temperature for 22 hours. With respect to the resulting reaction solution, the product was analyzed by high-performance liquid chromatography. Consequently, N-formyl-L-leucine was formed in an amount of 1.27 g (79.8% based on L-leucine).

### <Example 34>

1.31 g (0.01 mol) of L-leucine, 0.64 g (0.011 mol) of 29% aqueous ammonia, 1.80 g (0.03 mol) of methyl formate and 1.2 ml of water were added to a reaction vessel, and reacted under ice cooling for 2 hours and then at room temperature for 22 hours. With respect to the resulting reaction solution, the product was analyzed by high-performance liquid chromatography. Consequently, N-formyl-L-leucine was formed in an amount of 1.21 g (76.0% based on L-leucine).

### <Example 35>

19.7 g (0.15 mol) of L-leucine and 6.0 g (0.15 mol) of sodium hydroxide were added to 30 ml of water in a reaction vessel, and the solution was cooled to 0°C. 27.3 g (0.45 mol) of methyl formate was added dropwise thereto over a period of 9 hours, and the mixture was then vigorously stirred at room temperature for 15 hours. With respect to the resulting reaction solution, the product was analyzed by high-performance liquid chromatography. Consequently, N-formyl-L-leucine was formed in an amount of 19.2 g (80.4% based on L-leucine).

As is clear from the foregoing description, N-formylaspartic acid (or its salt) can be produced at good efficiency, and F-APM as a precursor of aspartame can be produced at a high purity in a high yield by the enzyme condensation reaction of this N-formylaspartic acid (or its salt) with L- and/or DL-phenylalanine methyl ester and the subsequent separation method. Aspartame can be produced in a high yield by deformylation of the thus-produced F-APM in the foregoing usual manner.

Further, the neutral amino acid can be subjected to N-formylation in quite a high yield.

Incidentally, in the formylation reaction, racemization hardly occurs during the reaction, and by using optically active aspartic acid and neutral amino acid (or their salts) as a starting material, an N-formylamino acid or its salt can be obtained as an optically active substance after the N-formylation reaction.

### Effects of the invention

According to the present invention, an N-formylamino acid, especially N-formylaspartic acid (or its salt) or an N-formyl-neutral-amino acid such as leucine (or its salt) which is important as various synthetic intermediates, particularly an intermediate for synthesis of a peptide or the like can be produced at good efficiency and industrially easily.

Further, it is also possible to produce, at good efficiency and industrially easily, N-formyl-α-L-aspartyl-L-phenylalanine methyl ester as a precursor of a sweetener aspartame from N-formyl-L-aspartic acid, and the aspartame.

Accordingly, the present invention is industrially quite useful especially in the field of foods and in the field of pharmaceuticals.

## Claims

1. A process for producing N- formylaspartic acid or its salt, **characterized by** reacting aspartic acid with formamide and/or methyl formate in the presence of a base.

2. A process for producing N- formylaspartic acid or its salt, **characterized by** reacting a salt of aspartic acid with formamide and/or methyl formate.

3. A process for producing aspartame, **characterized by** obtaining N-formyl-L-aspartic acid or its salt according to the process as claimed in claim 1 or 2, then condensing the N-formyl-L-aspartic acid or its salt with L-phenylalanine methyl ester or its salt for conversion to N-formyl-α-L-aspartyl-L-phenylalanine methyl ester, and then removing the formyl group therefrom.

4. A process for producing N-formyl-α-L-aspartyl-L-phenylalanine methyl ester, **characterized in that** in an enzyme condensation reaction of N-formyl-L-aspartic acid or its salt with L- and/or DL-phenylalanine methyl ester or their/its salt(s), a concentration of the N-formyl-L-aspartic acid in an aqueous solution is 1.2 mol/L or more.
The N-formyl-α-L-aspartyl-L-phenylalanine methyl ester may be in the form of an L- and/or D-phenylalanine methyl ester adduct.

5. A process for producing N-formyl-α-L-aspartyl-L-phenylalanine methyl ester, **characterized in that** in an enzyme condensation reaction of N-formyl-L-aspartic acid or its salt with L- and/or DL-phenylalanine methyl ester or their/its salt(s) after obtaining the N-formyl-L-aspartic acid or its salt according to the process as claimed in claim 1 or 2, a concentration of the N-formyl-L-aspartic acid in an aqueous solution is 1.2 mol/L or more.
The N-formyl-α-L-aspartyl-L-phenylalanine methyl ester may be in the form of an L- and/or D-phenylalanine methyl ester adduct.

6. A process for producing an L- and/or D-phenylalanine methyl ester adduct of N-formyl-α-L-aspartyl-L-phenylalanine methyl ester, **characterized in that** in an enzyme condensation reaction of N-formyl-L-aspartic acid or its salt with L- and/or DL-phenylalanine methyl ester or their/its salt(s), an enzyme condensation reaction during which the reaction solution becomes solid by the process of reaction is conducted in the presence of a trialkyl phosphate to render the reaction solution in a slurry state, and crystals of the L- and/or D-phenylalanine methyl ester adduct of N-formyl-α-L-aspartyl-L-phenylalanine methyl ester are separated from the slurry.

7. A process for producing an L- and/or D-phenylalanine methyl ester adduct of N-formyl-α-L-aspartyl-L-phenylalanine methyl ester, **characterized in that** in an enzyme condensation reaction of N-formyl-L-aspartic acid or its salt with L- and/or DL-phenylalanine methyl ester or their/its salt(s) after obtaining the N-formyl-L-aspartic acid or its salt according to the process as claimed in claim 1 or 2, an enzyme condensation reaction during which the reaction solution becomes solid by the process of reaction is conducted in the presence of a trialkyl phosphate to render the reaction solution in a slurry state, and crystals of the L- and/or D-phenylalanine methyl ester adduct of N-formyl-α-L-aspartyl-L-phenylalanine methyl ester are separated from the slurry.

8. A process for producing an L- and/or D-phenylalanine methyl ester adduct of N-formyl-α-L-aspartyl-L-phenylalanine methyl ester, **characterized in that** in an enzyme condensation reaction of N-formyl-L-aspartic acid or its salt with L- and/or DL-phenylalanine methyl ester or their/its salt(s), a concentration of N-formyl-L-aspartic acid in an aqueous solution is 1.2 mol/L or more, the reaction is conducted in the presence of a trialkyl phosphate, and crystals of the L- and/or D-phenylalanine methyl ester adduct of N-formyl-α-L-aspartyl-L-phenylalanine methyl ester are separated from the reaction solution in the slurry state.

9. A process for producing an L- and/or D-phenylalanine methyl ester adduct of N-formyl-α-L-aspartyl-L-phenylalanine methyl ester, **characterized in that** in an enzyme condensation reaction of N-formyl-L-aspartic acid or its salt with L- and/or DL-phenylalanine methyl ester or their/its salt(s) after obtaining the N-formyl-L-aspartic acid or its salt according to the process as claimed in claim 1 or 2, a concentration of N-formyl-L-aspartic acid in an aqueous solution is 1 . 2 mol/L or more, the reaction is conducted in the presence of a trialkyl phosphate, and crystals of the L- and/or D-phenylalanine methyl ester adduct of N-formyl-α-L-aspartyl-L-phenylalanine methyl ester are separated from the reaction solution in the slurry state.

10. A process for producing aspartame, **characterized by** obtaining N-formyl-α-L-aspartyl-L-phenylalanine methyl ester or the L- and/or D-phenylalanine methyl ester adduct of N-formyl-α-L-aspartyl-L-phenylalanine methyl ester according to the process as claimed in any one of claims 4 to 9, and then converting this to aspartame.

11. A process for producing N-formyl-α-L-aspartyl-L-phenylalanine methyl ester, **characterized by** suspending a phenylalanine methyl ester adduct of N-formyl-α-L-aspartyl-L-phenylalanine methyl ester in an aqueous solution having pH of from 1.0 to 4.5, and separating a solid.

12. A process for producing N-formyl-α-L-aspartyl-L-phenylalanine methyl ester, **characterized by** obtaining the phenylalanine methyl ester adduct of N-formyl-α-L-aspartyl-L-phenylalanine methyl ester according to the process as claimed in any one of claims 4 to 9, then suspending this adduct in an aqueous solution having pH of from 1.0 to 4.5, and separating a solid.

13. A process for producing aspartame, **characterized by** obtaining N-formyl-α-L-aspartyl-L-phenylalanine methyl ester according to the process as claimed in any one of claims 4 to 12, and then removing the formyl group therefrom.

14. A process for producing an N-formyl-neutral-amino acid or its salt, **characterized by** reacting a neutral amino acid with formamide and/or methyl formate in the presence of a base.

15. A process for producing an N-formyl-neutral-amino acid or its salt, **characterized by** reacting a salt of a neutral amino acid with formamide and/or methyl formate.
